# EUROPEAN PATENT APPLICATION

(11) **EP 4 098 200 A1**
(43) Date of publication of application: **07.12.2022**
(21) Application number: 21177524.2
(22) Date of filing: 03.06.2021
(51) Int. Cl.: A61B 6/03, A61B 6/00

(54) **PET IMAGING SYSTEM MOUNTING FRAME**

(71) Applicant: Koninklijke Philips N.V., 5656 AG Eindhoven (NL)
(72) Inventor: MUELHENS, Oliver, 5656 AE Eindhoven (NL); FRACH, Thomas, 5656 AE Eindhoven (NL)
(74) Representative: Philips Intellectual Property & Standards

(57) **Abstract**

A positron emission tomography imaging system (100) includes a plurality of gamma detector elements (130ᵢ), a plurality of detector cassettes (140ⱼ), and mounting frame (150). Each detector cassette (140ⱼ) is configured to replaceably receive a plurality of gamma detector elements (130i). The mounting frame (150) is configured to replaceably receive the detector cassettes (140ⱼ) at a plurality of angular positions (fₖ) around the axis (120) of the bore (110) such that at each angular position (fₖ) a plurality of detector cassettes (140ⱼ) are receivable along a direction parallel to the axis (120) of the bore (110), and a plurality of gamma detector elements (130ᵢ) are receivable within a cassette (140ⱼ) in a transaxial direction with respect to the axis (120) of the bore (110).

## Description

### TECHNICAL FIELD

The present disclosure relates to a positron emission tomography, PET, imaging system.

### BACKGROUND

PET imaging systems are used to study biological processes within the anatomy by means of a radiotracer. A radiotracer is typically attached to a molecule such as glucose or a ligand, and injected into the bloodstream of a subject. The bloodstream circulates the radiotracer within the anatomy, and the radiotracer is preferentially absorbed, or "uptaken" in certain regions depending on the region's biological function, and depending on the attachment molecule. PET images representing the spatial distribution of the radiotracer within the anatomy are then generated using the PET imaging system. A clinician may study such images in order to make a diagnosis of the subject.

The spatial distribution of a radiotracer within the anatomy is determined by detecting gamma quanta that are emitted by the radiotracer whilst it decays. As the radiotracer decays, it emits positrons. The positrons are annihilated locally by electrons, resulting in the simultaneous emission of pairs of oppositely-directed gamma quanta. The emission of each pair of gamma quanta may be referred-to as a radioactive decay event, or simply an "event".

In order to detect the gamma quanta, a PET imaging system includes multiple gamma detectors, often referred-to as "gamma detector elements". The gamma detector elements are arranged around a bore of the imaging system in order to detect the gamma quanta emitted from a portion of a subject within the bore. The gamma detector elements include scintillator arrays that are coupled to photodetector arrays. Each time a gamma quant is received by a gamma detector element, scintillation light is generated in its scintillator array, and the scintillation light is detected by its corresponding photodetector array. The detection times of the received gamma photons, often referred-to as "timestamps" and/or the light distribution generated in the scintillator array in response to the received gamma quanta, are referred-to as radioactive decay event data, or simply, "event data". The event data generated by the gamma detector elements in response to the received gamma quanta is processed in order to localize the origin of each event within the bore.

Processing the event data may involve an initial step of analyzing the scintillation light distribution that is detected by the photodetector array in response to each received gamma quant, and assigning the light distribution to a common received gamma quant; a process referred-to as "clustering". Clustering is performed in order to determine the most likely location on the detector at which each gamma quant was received and/or to determine the energy of the received gamma quant.

Processing the event data may also involve comparing the detection times of the received gamma photons in order to identify pairs of gamma quanta that are received within a predetermined time interval of one another; a process referred-to as "coincidence search". In coincidence search, pairs of gamma quanta that are received within a predetermined time interval of one another that is defined by the bore diameter and the speed of travel of a gamma photon, are assumed to have originated from a common radioactive decay event. Such pairs of gamma quanta are referred-to as "coincident pairs", and define a line of response, or "LOR" between the locations on the detectors at which they are detected. Under this assumption, a LOR intercepts the origin of the decay event, but its position along the LOR is uncertain. In so-called time-of-flight "TOF" PET imaging, a more accurate position of the actual decay event along the LOR may be determined based on a time difference between the times of detection of the gamma quanta in each coincident pair. The LORs from multiple decay events are used to reconstruct a PET image representing the distribution of the radiotracer in the subject.

The processing of event data in PET imaging systems is extremely intensive in view of the need to determine the event data for individual gamma quanta, and the rate at which the gamma quanta are received. The image quality of the resulting PET images is determined in part by the accuracy and the rate at which the origins of each coincident pair can be determined. Consequently, in PET imaging systems, the operations of coincidence search, and clustering, are typically performed by a common, central, processor.

The gamma detector elements in a PET imaging system are arranged around its bore by means of a mounting frame, or "gantry". The mounting frame directly, or indirectly supports the gamma detector elements. In conventional PET imaging systems, the geometry of the mounting frame, and consequently the axial field of view of the PET imaging system, is determined when the imaging system is constructed.

A document by Bruschini, C., et al, entitled "SPADnet: a fully digital, scalable, and networked photonic component for time-of-flight PET applications" published in Biophotonics: Photonic Solutions for Better Health Care IV, edited by Jürgen Popp, Valery V. Tuchin, Dennis L. Matthews, Francesco S. Pavone, Proc. of SPIE Vol. 9129, 912913 discloses a sensor tile interfaced to an FPGA-based PCB on its back. The resulting photonic module acts as an autonomous sensing and computing unit, individually detecting gamma photons as well as thermal and Compton events. It determines in real time basic information for each scintillation event, such as exact time of arrival, position and energy, and communicates it to its peers in the field of view. Coincidence detection therefore occurs directly in the ring itself, in a differed and distributed manner to ensure scalability. The selected true coincidence events are then collected by a snooper module, from which they are transferred to an external reconstruction computer using Gigabit Ethernet.

However, there remains room to improve the construction of PET imaging systems.

### SUMMARY

According to one aspect of the present disclosure, a PET imaging system, is provided. The PET imaging system includes a bore for receiving a subject. The bore includes an axis. The PET imaging system also includes a plurality of gamma detector elements, a plurality of detector cassettes, and mounting frame. Each gamma detector element includes a scintillator array coupled to a photodetector array. Each detector cassette is configured to replaceably receive a plurality of gamma detector elements. The mounting frame is configured to replaceably receive the detector cassettes at a plurality of angular positions around the axis of the bore such that at each angular position a plurality of detector cassettes are receivable along a direction parallel to the axis of the bore, and a plurality of gamma detector elements are receivable within a cassette in a transaxial direction with respect to the axis of the bore for detecting gamma quanta received from within the bore.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating, in orthogonal views, an example PET imaging system 100 including a plurality of detector cassettes 140ⱼ, in accordance with some aspects of the present disclosure.
Fig. 2 is a schematic diagram illustrating an example of a detector cassette 140ⱼ (A), a plurality of detector cassettes 140ⱼ coupled to a backplane 170 (B), and a section of a mounting frame 150 including a plurality of backplanes 170 (C), in accordance with some aspects of the present disclosure.
Fig. 3 is a schematic diagram illustrating an example PET imaging system 100 in which the processors within each detector cassette 140ⱼ include a first communication path 310ₖ coupling the processor to a processor at an adjacent angular position, and a second communication path 320ₖ coupling the processor to a processor at a non-adjacent angular position, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity.

In the following description, reference is made to a PET imaging system. It is to be appreciated that the PET imaging system may be any type of PET imaging system, including a time-of-flight "TOF" PET imaging system, and a non-TOF-PET imaging. Reference is made herein to examples of PET imaging systems that include multiple gamma detector elements. The gamma detector elements are supported directly or indirectly by a mounting frame and arranged to provide an axial field of view with a desired length. In this respect, it is to be appreciated that the length of the axial field of view may be sufficient to image a portion of a subject, such as the heart, the brain, the lungs, or a substantial portion, or even the entire length, of a human subject. Some examples of the present disclosure may therefore be used to provide so-called "full-body" PET imaging systems.

In the following description, reference is made to a processor. In some examples, the processor is included within a cassette. The processor carries out various methods, which may thus be referred-to as computer-implemented methods. In this respect, it is noted that the computer-implemented methods may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, the gamma detector elements in a PET imaging system are arranged around its bore by means of a mounting frame, often known as a gantry. The mounting frame directly, or indirectly supports the gamma detector elements. In conventional PET imaging systems, the geometry of the mounting frame, and consequently the axial field of view of the PET imaging system, is determined when the imaging system is constructed. A drawback of such systems is that in order to generate PET images that have an axial length that exceeds the axial field of view of the PET imaging system, a subject being examined must either be translated within the imaging system, or imaged in another PET imaging system. In-time, a PET imaging system with a fixed axial field of view may therefore become obsolete.

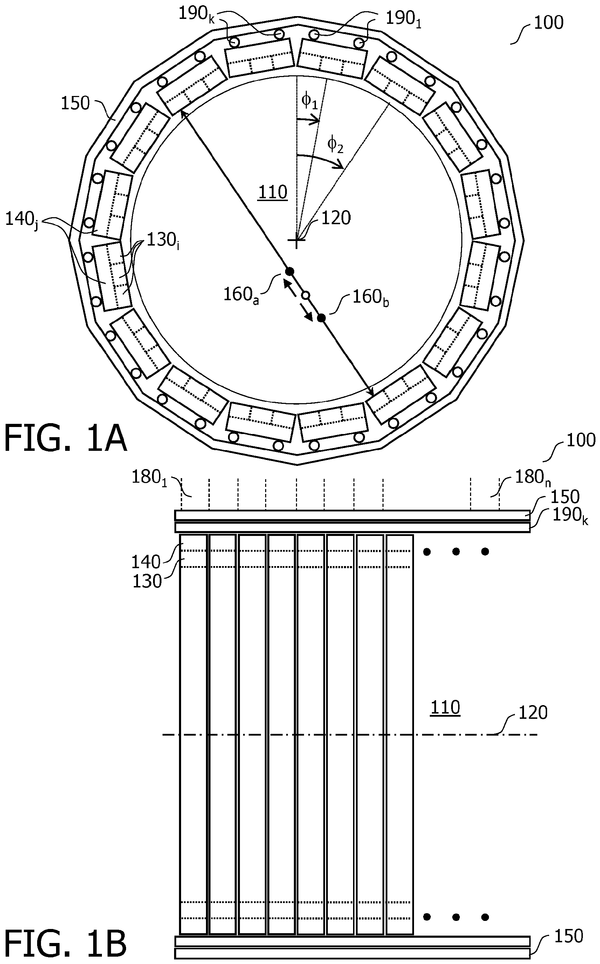

Fig. 1 is a schematic diagram illustrating, in orthogonal views, an example PET imaging system 100 including a plurality of detector cassettes 140ⱼ, in accordance with some aspects of the present disclosure. The positron emission tomography imaging system 100 includes a bore 110 for receiving a subject. The bore 110 has an axis 120. The axis 120 extends into the plane of the illustration. In-use, the bore 110 may receive a subject, such as a human body, along the axis 120, by means of a patient bed (not illustrated). The patient bed may be extended into the bore 110 in order to perform a PET imaging procedure on the subject. The PET imaging procedure may be used to detect a distribution of a radiotracer within a region of interest in the subject. A radiotracer such as ¹⁸F-fluoro-2-deoxy-D-glucose "FDG", or another radiotracer, may have been previously injected into the subject for this purpose. The region of interest may be a particular organ, such as the brain, the lungs, and so forth, or a substantial portion of the body in a so-called "full-body" PET scan.

The PET imaging system 100 illustrated in Fig. 1 also includes a plurality of gamma detector elements 130i, a plurality of detector cassettes 140ⱼ, and a mounting frame 150. Each gamma detector element 130ᵢ includes a scintillator array (not illustrated), and which is coupled to a photodetector array (not illustrated). The gamma detector elements 130ᵢ generate event data in response to received gamma quanta, such as the gamma quanta 160ₐ, 160_{b} illustrated in Fig. 1A. The event data represent detection times of the received gamma quanta 160ₐ, 160_{b} and/or a light distribution generated in the scintillator array in response to the received gamma quanta 160ₐ, 160_{b}. The scintillator array may be formed from various scintillator materials that generate scintillation light in response to received gamma quanta. Scintillator materials such as lutetium yttrium orthosilicate "LYSO", bismuth germanate "BGO", and garnets such as gadolinium aluminium gallium garnet "GAGG", and so forth, are known for this purpose. The photodetector array may be formed from various materials that are suitable for detecting the scintillation light. A silicon photodetector array that includes a plurality of silicon avalanche photodiodes may for example be used for this purpose. The photodetector array is optically coupled to the scintillator array such that the spatial distribution of the scintillation light that is generated in the scintillator array in response to received gamma quanta, is measured by the photodetector array. The photodetector array may be coupled to readout electronics for generating the event data from electrical signals generated by the photodetector array. The gamma detector elements may be supplied with a clock signal for synchronizing the event data with the event data generated by other gamma detector elements, thereby providing accurate measurements of the detection times.

Each detector cassette 140ⱼ illustrated in Fig. 1 is configured to replaceably receive a plurality of gamma detector elements 130ᵢ. Moreover, the mounting frame 150 is configured to replaceably receive the detector cassettes 140ⱼ at a plurality of angular positions fₖ around the axis 120 of the bore 110 such that at each angular position fₖ a plurality of detector cassettes 140ⱼ are receivable along a direction parallel to the axis 120 of the bore 110, and a plurality of gamma detector elements 130ᵢ are receivable within a cassette 140ⱼ in a transaxial direction with respect to the axis 120 of the bore 110 in order to detect gamma quanta 160ₐ, 160_{b} received from within the bore 110.

Since the detector cassettes 140ⱼ can replaceably receive the gamma detector elements 130i, and the mounting frame 150 can replaceably receive the detector cassettes, the PET imaging system may be constructed with a desired number of detector cassettes 140ⱼ along a direction parallel to the axis 120 of the bore 110, and with a desired number of gamma detector elements 130ᵢ in a transaxial direction with respect to the axis 120 of the bore 110. This provides the ability to adapt the axial field of view of the PET imaging system as desired, as well as the ability to adapt the resolution of PET images generated by the PET imaging system, respectively. The PET imaging system may therefore be adapted, or upgraded, thereby obviating the risk that the imaging system is rendered obsolete over time. The replaceability of the gamma detector elements and the detector cassettes also facilitates maintenance of the PET imaging system.

Various mechanical and/or electrical couplings are contemplated for facilitating the gamma detector elements, and the detector cassettes, to be replaceably received by the detector cassettes and the mounting frame, respectively. Mechanical couplings such as screws, bolts, latches, clips, catches, magnets, and so forth may be used, for example. Electrical couplings, or connectors, card edge connectors, DIN connectors, backplane connectors. Mezzanine connectors, D-sub connectors, POGO connectors, PCI card connectors, and so forth may be used, for example. Various materials are contemplated for use in the mounting frame 150. For example, mounting frame 150 may be formed from a metal such as steel, or aluminium.

The PET imaging system 100 described above may include one or more additional features. These are described below with reference to further examples. It is noted that whilst the examples may be described individually, these examples may also be combined to provide further advantageous effects.

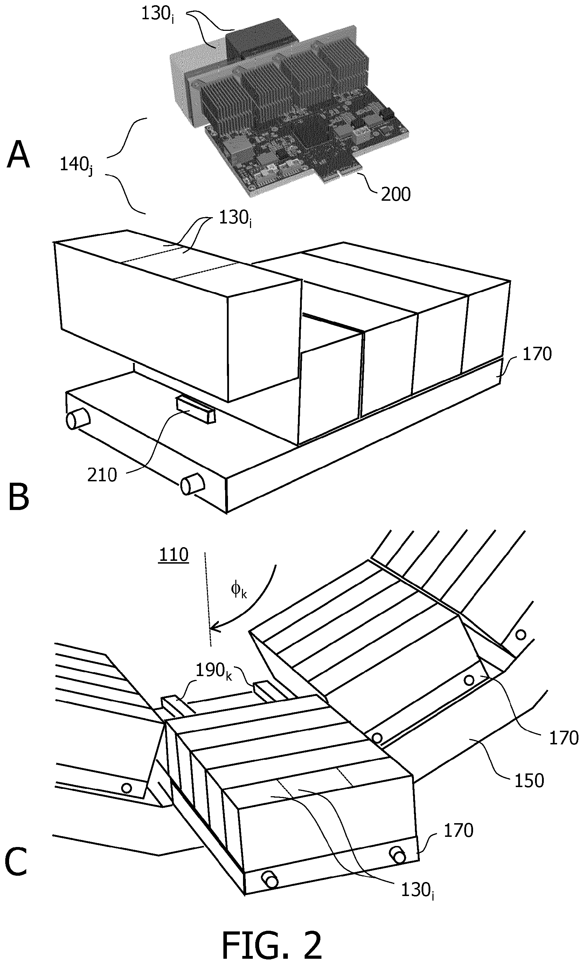

In one example, the detector cassettes 140ⱼ are received by backplanes. Fig. 2 is a schematic diagram illustrating an example of a detector cassette 140ⱼ (A), a plurality of detector cassettes 140ⱼ coupled to a backplane 170 (B), and a section of a mounting frame 150 including a plurality of backplanes 170 (C), in accordance with some aspects of the present disclosure. In this example, the mounting frame 150 comprises a backplane 170 at each angular position fₖ. The backplane 170 at each angular position fₖ comprises a plurality of mechanical couplings configured to replaceably receive the plurality of detector cassettes 140ⱼ at discrete axial positions 180_{1..n} along the direction parallel to the axis of the bore. The mechanical couplings are not illustrated in Fig. 2, but may be provided by one of the aforementioned mechanical couplings such as screws, bolts, and so forth. The mechanical couplings may be disposed between a detector cassette 140j and the backplane 170 in Fig. 2B, for example. The discrete axial positions facilitate accurate and repeatable alignment of the detector cassettes, and ultimately the gamma detector elements, with respect to the bore 110.

In one example, the backplane 170 provides a power supply for the one or more detector cassettes 140ⱼ that are received at each angular position fₖ.

In one example, the mounting frame 150 includes a translation mechanism 190ₖ at each angular position fₖ around the axis of the bore. The translation mechanism 190ₖ at each angular position fₖ is configured to position the plurality of detector cassettes 140ⱼ along the direction parallel to the axis 120 of the bore 110. This is illustrated in Fig. 2C, wherein an example drawer-type translation mechanism is provided that includes a pair of parallel rails at each angular position fₖ. An alternative translation mechanism 190ₖ is illustrated in Fig. 1A. This latter translation mechanism includes a pair of parallel cylindrical rods at each angular position fₖ. Other types of translation mechanisms that use wheels, bearings, gears, and so forth may be used in a similar manner to position the detector cassettes 140ⱼ along the direction parallel to the axis 120 of the bore 110. The use of such translation mechanisms facilitates the adaptation, and also the maintenance of the PET imaging system 100.

In one example, each backplane 170 includes a transceiver. The transceiver is configured to control a communication of event data generated by the gamma detector elements 130ᵢ between different cassettes 140ⱼ.

In one example, each detector cassette 140ⱼ comprises a processor configured to receive event data generated by the one or more gamma detector elements 130ᵢ received in the detector cassette in response to received gamma quanta. The event data represents detection times of the received gamma quanta and/or a light distribution generated in the scintillator arrays of the one or more gamma detector elements 130ᵢ in response to the received gamma quanta. In this example, the processor is further configured to:
cluster the event data by assigning the light distribution generated in one or more scintillator arrays to a common received gamma quant; and/or
identify coincident pairs of received gamma quanta having detection times within a predetermined time interval of one another.

In comparison to a conventional PET processing architecture that uses a central processor to process the event data generated by the gamma detector elements, i.e. the detection times of the received gamma quanta 160ₐ, 160_{b} and/or a light distribution generated in the scintillator array in response to the received gamma quanta 160ₐ, 160_{b}, the distributed processing architecture provided by the gamma detector elements 130ᵢ, and in which processing of the event data takes place locally with respect to the detector elements, alleviates the problem that a central processing unit has to be capable of handling the combined data rate arising from the events from all gamma detector elements. This distributed processing architecture therefore permits the use of lower speed processors for processing the event data because the individual processors may operate temporally in parallel.

The processors may cluster the event data by assigning the light distribution generated in the one or more scintillator arrays to a common received gamma quant. This operation may be carried out based on the detection times of portions of the light distribution by the photodetectors in the array, together with the expected lateral spread of the light distribution from a gamma quant. Alternatively of additionally, the processors may compute a total energy of the received gamma quant based on the cluster. This operation may be carried out by integrating the light distribution for the cluster, or counting the individual number scintillation light photons that are generated. The photodetector array may include an electrical integrating circuit, or a so-called photon counting detector, for these purposes respectively. Alternatively or additionally, the processors may determine a position of the received gamma quant based on the cluster. The processor may determine the position of the received gamma quant 160ₐ, 160_{b} by computing the centroid of the light distribution for a cluster, for example.

In one example, the total energy of the gamma quant may be compared to an expected energy of the gamma quanta emitted by the radiotracer during its decay in order to distinguish between true coincidence events, and scattered coincidence events. If the total energies of both gamma quanta in the coincident pair are within a predetermined range of the expected energy, the coincident pair may be labelled as a "true coincidence" event. If the total energy of one or both gamma quanta in the coincident pair are outside the predetermined range, the coincident pair may be labelled respectively as a "scattered coincidence" event or a "scatter event". Scatter events may be used to correct the true coincidence events, or omitted from use in PET image reconstruction.

The processor may identify coincident pairs of received gamma quanta having detection times within a predetermined time interval of one another by:
comparing the detection time of a gamma quant detected by gamma detector element 130ᵢ of the detector cassette, with the detection times of one or more other gamma quanta detected by other gamma detector elements 130ᵢ of other detector cassettes 140ⱼ to identify a corresponding gamma quant having a detection time within the predetermined time interval.

The processor may further:
identify, based on the comparing, a corresponding processor of the gamma detector element 130ᵢ detecting the corresponding gamma quant; and
transmit the event data of the detected gamma quant to the corresponding processor, and/or receive the event data of the corresponding gamma quant from the corresponding processor.

Providing this functionality at the locations of the gamma detector elements, rather than in a common, central processor for the PET imaging system, allows the axial field of view of the PET imaging system to be extended without being limited by the processing power of a common central processor for performing these operations.

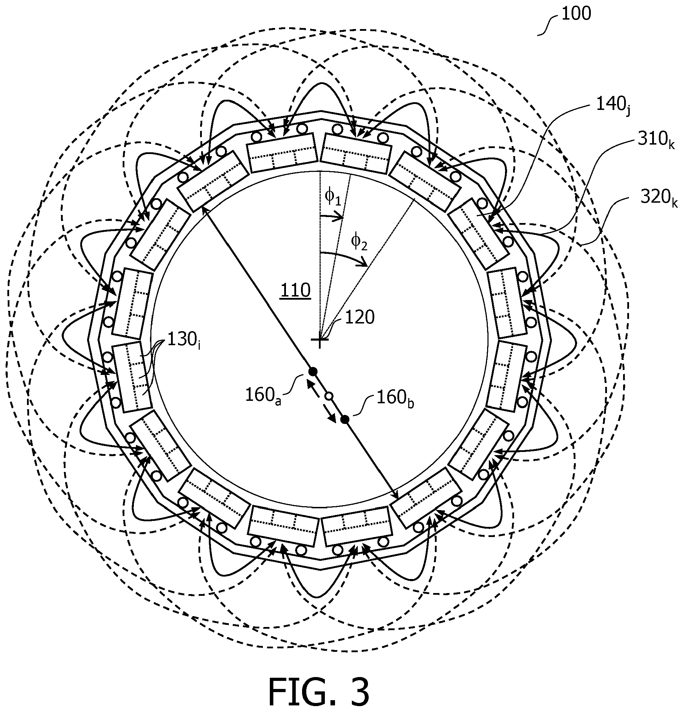

In one example, communication paths are provided between the processors to facilitate the transferring of event data between the processors. Fig. 3 is a schematic diagram illustrating an example PET imaging system 100 in which the processors within each detector cassette 140ⱼ include a first communication path 310ₖ coupling the processor to a processor at an adjacent angular position, and a second communication path 320ₖ coupling the processor to a processor at a non-adjacent angular position, in accordance with some aspects of the present disclosure. In this example, each processor comprises a first communication path 310ₖ coupling the processor to a processor at an adjacent angular position, and a second communication path 320ₖ coupling the processor to a processor at a non-adjacent angular position.

The first and second communication paths 310ₖ and 320ₖ may in general be electrical or optical communication paths. The angular positions are represented by the angle fₖ in Fig. 3, and which corresponds to the angle fₖ in Fig. 1. Thus, as illustrated by the example in Fig. 3, the processor in each detector cassette 140ⱼ has a first communication path 310ₖ to a processor in a detector cassette at an adjacent angular position, and a second communication path 320ₖ to a processor in detector cassette at a non-adjacent angular position. The non-adjacent detector cassette may in general be two or more detector cassettes away in the circumferential direction, i.e. the closest non-adjacent detector cassette to the detector cassette 140₁ would be two angular positions away, i.e. detector cassette 140₃.

Each processor is also configured to transmit the event data generated by the gamma detector elements 130ᵢ at its angular position fₖ to the processor at the adjacent angular position, and to the processor at a non-adjacent angular position, via the first communication path 310ₖ, and the second communication path 320ₖ, respectively; and/or each processor is configured to receive from the processor at the adjacent angular position, the event data generated by the gamma detector elements 130ᵢ at the adjacent angular position, and to receive from the processor at the non-adjacent angular position the event data generated by the gamma detector elements 130ᵢ at the non-adjacent angular position, via the first communication path 310ₖ, and via the second communication path 320ₖ, respectively.

The first communication paths 310ₖ permit event data to be transferred between the processors of adjacent detector cassettes. The second communication paths 320ₖ permit the transfer of event data between the processors of detector cassettes that are more separated around the bore 110. Both communication paths may also be used to exchange handshake data. In PET imaging systems, the coincident pairs of oppositely-directed gamma quanta are typically detected on opposite sides of the bore 110. The second communication paths 320ₖ therefore provide faster, i.e. reduced latency, transfer of the event data around the bore 110 between the expected detection positions of coincident gamma. By obviating the need to transfer event data around the bore via every single detector cassette along the path, improved PET imaging system performance, is provided. In-use, the processors may dynamically select which of the first and second communication paths to use when communicating the event data. The path may for example be selected based on the shortest distance between the origin and the destination of the event data, or the current utilization of the available communication paths.

In the arrangement illustrated in Fig. 2, the detector cassettes 140ⱼ may also be electrically coupled to backplanes at each angular position. Thus, in one example, the mounting frame 150 comprises a backplane 170 at each angular position fₖ. The backplane 170 at each angular position fₖ comprises one or more optical or electrical connectors 200 that are configured to couple to one or more corresponding optical or electrical connectors 210 disposed on the one or more detector cassettes 140ⱼ received at the angular position fₖ. Moreover, the optical or electrical connectors 200, 210 comprise one or more optical or electrically conductive paths configured to provide the first communication path 310ₖ and the second communication path 320ₖ for each processor.

In this example, suitable electrical connectors include card edge connectors, DIN connectors, backplane connectors, Mezzanine connectors, D-sub connectors, POGO connectors, PCI card connectors, and so forth. Suitable optical connectors include SC, FC, LC, ST, MU, E2000, MTRJ, Opti-Jack and FDDI connectors.

In the arrangement illustrated in Fig. 2, the backplane 170 at each angular position fₖ may include one or more inter-backplane optical or electrical connectors that couple to one or more corresponding inter-backplane optical or electrical connectors disposed on the mounting frame at the angular position fₖ. These inter-backplane connectors are not illustrated in Fig. 2. The one or more inter-backplane connectors at each angular position fₖ couple to the one or more corresponding inter-backplane connectors at the angular position fₖ along a direction parallel to the axis 120. In so doing, the inter-backplane connectors at each angular position fₖ may be coupled to and de-coupled from the corresponding inter-backplane connectors at the angular position fₖ by translating the backplane 170 along a direction parallel to the axis 120. The backplane 170 may be translated by means of the translation mechanism 190ₖ described above, as illustrated in Fig. 2. The corresponding inter-backplane connectors include optical or electrically conductive paths that provide the first communication path 310ₖ and the second communication path 320ₖ between the processors on different backplanes. In so doing, the first and second communication paths 310ₖ, 310ₖ may be provided between different backplanes in the manner illustrated in Fig. 3. Moreover, the ability to couple and de-couple the inter-backplane connectors by translating the backplane along a direction parallel to the axis 120, facilitates the replacement of the gamma detector elements (130ᵢ) and the detector cassettes (140ⱼ) without disturbing the optical or electrically conductive paths.

Thus, in one example, the mounting frame 150 includes a backplane 170 at each angular position fₖ. The backplane 170 at each angular position fₖ includes one or more inter-backplane optical or electrical connectors configured to couple to one or more corresponding inter-backplane optical or electrical connectors disposed on the mounting frame at the angular position fₖ. The one or more inter-backplane optical or electrical connectors at each angular position fₖ are configured to couple to the one or more corresponding inter-backplane optical or electrical connectors at the angular position fₖ along a direction parallel to the axis 120. Moreover, the one or more corresponding inter-backplane optical or electrical connectors disposed on the mounting frame at the angular positions fₖ are coupled to one another via one or more optical or electrically conductive paths for providing the first communication path 310ₖ and the second communication path 320ₖ between the processors on different backplanes.

In this example, suitable electrical connectors include card edge connectors, DIN connectors, backplane connectors, Mezzanine connectors, D-sub connectors, POGO connectors, PCI card connectors, and so forth. Suitable optical connectors include SC, LC, MU, E2000, MTRJ, Opti-Jack, and FDDI connectors. Other types of connectors that may be coupled and de-coupled using a similar push-pull action may also be used.

The above examples are to be understood as illustrative of the present disclosure, and not restrictive. Further examples are also contemplated. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A positron emission tomography imaging system (100) comprising:
a bore (110) for receiving a subject, the bore (110) comprising an axis (120);
a plurality of gamma detector elements (130i);
a plurality of detector cassettes (140ⱼ); and
mounting frame (150);
wherein each gamma detector element (130ᵢ) comprises a scintillator array coupled to a photodetector array;
wherein each detector cassette (140ⱼ) is configured to replaceably receive a plurality of gamma detector elements (130i);
wherein the mounting frame (150) is configured to replaceably receive the detector cassettes (140ⱼ) at a plurality of angular positions (fₖ) around the axis (120) of the bore (110) such that at each angular position (fₖ) a plurality of detector cassettes (140ⱼ) are receivable along a direction parallel to the axis (120) of the bore (110), and a plurality of gamma detector elements (130ᵢ) are receivable within a cassette (140ⱼ) in a transaxial direction with respect to the axis (120) of the bore (110) for detecting gamma quanta (160ₐ, 160_{b}) received from within the bore (110).

2. The positron emission tomography imaging system according to claim 1, wherein the mounting frame (150) comprises a backplane (170) at each angular position (fₖ); and wherein the backplane (170) at each angular position (fₖ) comprises a plurality of mechanical couplings configured to replaceably receive the plurality of detector cassettes (140ⱼ) at discrete axial positions (180_{1..n}) along the direction parallel to the axis of the bore.

3. The positron emission tomography imaging system according to claim 2, wherein the backplane (170) is further configured to provide a power supply for the one or more detector cassettes (140ⱼ) received at each angular position (fₖ).

4. The positron emission tomography imaging system according to claim 2 or claim 3, wherein the mounting frame (150) comprises a translation mechanism (190ₖ) at each angular position (fₖ) around the axis of the bore; and wherein the translation mechanism (190ₖ) at each angular position (fₖ) is configured to position the plurality of detector cassettes (140ⱼ) along the direction parallel to the axis (120) of the bore (110).

5. The positron emission tomography imaging system according to claim 2, wherein each backplane (170) comprises a transceiver configured to control a communication of event data generated by the gamma detector elements (130ᵢ) between different cassettes (140ⱼ).

6. The positron emission tomography imaging system according to claim 1, wherein each detector cassette (140ⱼ) comprises a processor configured to receive event data generated by the one or more gamma detector elements (130ᵢ) received in the detector cassette in response to received gamma quanta, the event data representing detection times of the received gamma quanta and/or a light distribution generated in the scintillator arrays of the one or more gamma detector elements (130ᵢ) in response to the received gamma quanta; and wherein the processor is further configured to:
cluster the event data by assigning the light distribution generated in one or more scintillator arrays to a common received gamma quant; and/or
identify coincident pairs of received gamma quanta having detection times within a predetermined time interval of one another.

7. The positron emission tomography imaging system according to claim 6, wherein the processor is configured to cluster the event data by assigning the light distribution generated in the one or more scintillator arrays to a common received gamma quant; and/or
wherein the processor is further configured to compute a total energy of the received gamma quant based on the cluster; and/or
wherein the processor is further configured to determine a position of the received gamma quant based on the cluster.

8. The positron emission tomography imaging system according to claim 6, wherein the processor is configured to identify coincident pairs of received gamma quanta having detection times within a predetermined time interval of one another by:
comparing the detection time of a gamma quant detected by gamma detector element (130ᵢ) of the detector cassette, with the detection times of one or more other gamma quanta detected by other gamma detector elements (130ᵢ) of other detector cassettes (140ⱼ) to identify a corresponding gamma quant having a detection time within the predetermined time interval.

9. The positron emission tomography imaging system according to claim 8, wherein the processor is further configured to:
identify, based on the comparing, a corresponding processor of the gamma detector element (130ᵢ) detecting the corresponding gamma quant; and to
transmit the event data of the detected gamma quant to the corresponding processor, and/or receive the event data of the corresponding gamma quant from the corresponding processor.

10. The positron emission tomography imaging system according to claim 6, wherein each processor comprises a first communication path (310ₖ) coupling the processor to a processor at an adjacent angular position, and a second communication path (320ₖ) coupling the processor to a processor at a non-adjacent angular position; and
wherein each processor is configured to transmit the event data generated by the gamma detector elements (130ᵢ) at its angular position (fₖ) to the processor at the adjacent angular position, and to the processor at a non-adjacent angular position, via the first communication path (310ₖ), and the second communication path (320ₖ), respectively; and/or
wherein each processor is configured to receive from the processor at the adjacent angular position, the event data generated by the gamma detector elements (130ᵢ) at the adjacent angular position, and to receive from the processor at the non-adjacent angular position the event data generated by the gamma detector elements (130ᵢ) at the non-adjacent angular position, via the first communication path (310ₖ), and via the second communication path (320ₖ), respectively.

11. The positron emission tomography imaging system according to claim 10, wherein the mounting frame (150) comprises a backplane (170) at each angular position (fₖ);
wherein the backplane (170) at each angular position (fₖ) comprises one or more optical or electrical connectors (200) configured to couple to one or more corresponding optical or electrical connectors (210) disposed on the one or more detector cassettes (140ⱼ) received at the angular (fₖ) position; and
wherein the optical or electrical connectors (200, 210) comprise one or more optical or electrically conductive paths configured to provide the first communication path (310ₖ) and the second communication path (320ₖ) for each processor.

12. The positron emission tomography imaging system according to claim 10 or claim 11, wherein the mounting frame (150) comprises a backplane (170) at each angular position (fₖ);
wherein the backplane (170) at each angular position (fₖ) comprises one or more inter-backplane optical or electrical connectors configured to couple to one or more corresponding inter-backplane optical or electrical connectors disposed on the mounting frame at the angular position (fₖ);
wherein the one or more inter-backplane optical or electrical connectors at each angular position (fₖ) are configured to couple to the one or more corresponding inter-backplane optical or electrical connectors at the angular position (fₖ) along a direction parallel to the axis (120); and
wherein the one or more corresponding inter-backplane optical or electrical connectors disposed on the mounting frame at the angular positions (fₖ) are coupled to one another via one or more optical or electrically conductive paths for providing the first communication path (310ₖ) and the second communication path (320ₖ) between the processors on different backplanes.
